(19) Europäisches Patentamt
European Patent Office
Office européen des brevets

(11) Veröffentlichungsnummer: **0 019 281**
**B1**

# (12) EUROPÄISCHE PATENTSCHRIFT

(45) Veröffentlichungstag der Patentschrift: **29.12.82**

(51) Int. Cl.[3]: **C 07 C 76/06**, C 07 C 79/12

(21) Anmeldenummer: **80102695.6**

(22) Anmeldetag: **15.05.80**

## (54) Verfahren zur Entfernung von Nitrosierungsmittel(n) aus nitrierten aromatischen Verbindungen.

(30) Priorität: **21.05.79 DE 2920448**
**04.03.80 DE 3008243**

(43) Veröffentlichungstag der Anmeldung:
**26.11.80 Patentblatt 80/24**

(45) Bekanntmachung des Hinweises auf die Patenterteilung:
**29.12.82 Patentblatt 82/52**

(84) Benannte Vertragsstaaten:
**CH DE FR GB IT LI NL**

(56) Entgegenhaltungen:
**DE-A-2 831 119**
**DE-A-2 835 530**
**DE-A-2 840 551**
**Chemical Abstracts Band 75, Nr. 13, 27. September 1971 Columbus, Ohio, USA J. KOVACICOVA et al. »Laboratory purification of fenitrothion« Seite 265, rechte Spalte, Abstract Nr. 87 406 g.**
**Chemical Abstracts Band 73, Nr. 12, 21. September 1970 Columbus, Ohio, USA G. EISENBRAND et al. »Trace analysis of N-nitroso compounds. II. Steam distillation at neutral, alkaline, and acid pH under reduced and atmospheric pressure« Seite 465, linke Spalte, Abstract Nr. 62 440 j**

(73) Patentinhaber: **HOECHST AKTIENGESELLSCHAFT, Postfach 80 03 20, D-6230 Frankfurt/Main 80 (DE)**

(72) Erfinder: **Habig, Kurt, Dr., Hundertmorgenring 98, D-6082 Mörfelden (DE)**
Erfinder: **Baessler, Konrad, Dr., Drosselweg 1, D-6230 Frankfurt am Main 80 (DE)**
Erfinder: **Schulz, Lothar, Dr., Bergstrasse 40, D-6239 Eppstein/Taunus (DE)**
Erfinder: **Schütte, Heinz, Dr., Ostpreussenstrasse 83, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verfahren zur Entfernung von Nitrosierungsmittel(n) aus nitrierten aromatischen Verbindungen

Unter »nitrierten aromatischen Verbindungen« werden im folgenden aromatische Nitroverbindungen verstanden, welche durch Nitrierung entsprechender aromatischer Ausgangsverbindungen mit konzentrierter Salpetersäure oder mit Nitriersäure (konzentrierte Salpetersäure/Schwefelsäure) und übliche Aufarbeitung hergestellt worden sind. Aromatische Nitroverbindungen, welche noch durch auf die Nitrierung folgende Reaktionsschritte weiter umgesetzt wurden, sollen unter »nitrierten aromatischen Verbindungen« hier nicht verstanden werden.

Nitrierte aromatische Verbindungen sind End- und Zwischenprodukte auf verschiedenen Sachgebieten, insbesondere auf dem Sprengstoff, Pharma- und Pflanzenschutzsektor.

Eine als Zwischenprodukt auf dem Pflanzenschutzsektor wichtige nitrierte aromatische Verbindung ist z. B. das durch Nitrierung von p-Chlor-benzotrifluorid erhältliche 4-Chlor-3,5-dinitrobenzotrifluorid, welches u. a. durch Umsetzung mit Di-n-propylamin das Herbizid Trifluralin (= 4-Di-n-propylamino-3,5-dinitrobenzotrifluorid) liefert. Diese Synthese läßt sich durch folgendes Reaktionsschema wiedergeben:

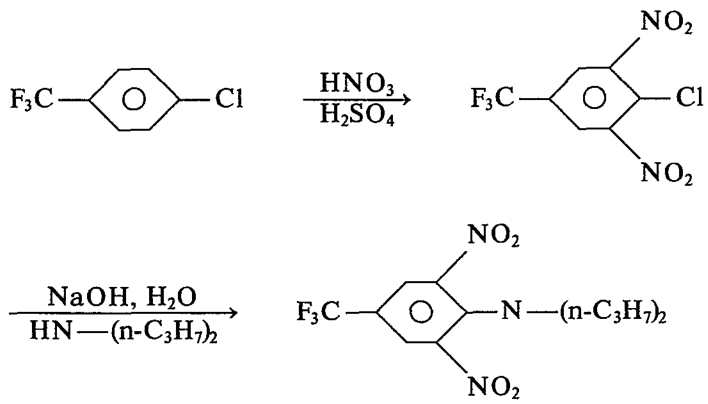


Mit Hilfe moderner Analysemethoden hat man in dem so hergestellten Trifluralin nun jedoch geringe Mengen von Nitrosaminen gefunden, die zumindest bei Tieren karzinogen wirken können und deswegen außerordentlich unerwünscht sind (siehe z. B. DE-OS 2 835 530). Die Bildung dieser Nitrosamine dürfte auf der Nitrosierung des in der letzten Reaktionsstufe eingesetzten Di-n-propylamins durch Nitrosierungsmittel beruhen, die in der Vorstufe des Trifluralins — im 4-Chlor-3,5-dinitrobenzotrifluorid — von der Nitrierungsreaktion her in geringerer Menge vorhanden zu sein scheinen.

Man hat daher bereits verschiedene Verfahren entwickelt, um die Nitrosamine aus dem Trifluralin wieder so vollständig wie möglich zu entfernen. Nach dem Verfahren z. B. der DE-OS 2 831 119 geschieht dies durch Behandlung mit 20—38%iger Salzsäure oder mit gasförmigem Chlorwasserstoff in flüssiger Phase bei Temperaturen bis zu 140° C.

Eine Behandlung mit Brom, Chlor, N-Bromsuccinimid, N-Chlorsuccinimid, Bromchlorid, Pyridinperbromid und/oder Pyridiniumbromidperbromid zum Zwecke ebenfalls der Verminderung der Nitrosaminkonzentration wird in der DE-OS 2 835 530 und eine Behandlung mit Phosphor- oder Schwefelhalogeniden und -oxihalogeniden oder mit $TiCl_4$ in der DE-OS 2 837 529 beschrieben.

Diese Methoden führen zunächst zu einem befriedigenden Abfall der Nitrosaminwerte, doch sollen diese Werte bei längerer Reaktionszeit wieder ansteigen; wenn die Reaktionszeit daher nicht genau optimiert wird, kann durchaus der Fall eintreten, daß die nach diesen Verfahrensweisen an sich möglichen niedrigen Nitrosaminwerte nicht erhalten werden. Außerdem bedeuten diese Verfahrensweisen einen zusätzlichen Arbeitsgang mit der Notwendigkeit der Aufarbeitung und gegebenenfalls Rückgewinnung der eingesetzten Reagentien.

Ein rein analytisches Verfahren, bei welchem Spuren von Nitrosaminen aus dem jeweiligen Substrat in neutralem, alkalischem oder saurem Milieu durch eine Wasserdampfdestillation entfernt und dann quantitativ bestimmt werden, ist beschrieben in dem Artikel »Trace Analysis of N-Nitroso-Compounds« von G. Eisenbrand et al. in Fresenius' Zeitschrift für analytische Chemie 1970, Bd. 251, S. 22 bis 24, referiert in C. A. 73 (1970) 465, abstr. 62440j. Durch das Verfahren lassen sich aber nur verschiedene relativ niedermolekulare Nitrosamine quantitativ entfernen und damit auch quantitativ bestimmen; insbesondere bei Nitrosaminen mit mehr als etwa 12 bis 16 C-Atomen funktioniert das Verfahren nicht mehr.

Man hat auch schon versucht, es gar nicht erst zur Bildung der Nitrosamine kommen zu lassen, indem man aus der nitrierten Vorstufe des Trifluralins — dem 4-Chlor-3,5-dinitrobenzotrifluorid — das darin als Nebenprodukt enthaltene Nitrosierungsmittel soweit wie möglich entfernt (DE-OS 2 840 551).

Dies geschieht durch eine Behandlung des genannten Vorsprodukts

a) mit einer wäßrigen Lösung einer anorganischen Base von einer Temperatur von 50 bis 100°C und — gegebenenfalls nach Abtrennen des so behandelten Produkts —
b) Hindurchleiten eines Inertgases (z. B. Luft) durch eine wäßrige Mischung des Produkts bei der gleichen Temperatur und
c) gegebenenfalls Wiederholung der Stufen a und/oder b beliebige Male.

Durch diese Verfahrensweise wird der Gehalt an Nitrosierungsmittel(n) in dem 4-Chlor-3,5-dinitrobenzotrifluorid offensichtlich derart erniedrigt, daß bei der nachfolgenden Umsetzung mit Di-n-propylamin in dem dann erhaltenen Trifluralin ähnlich niedrige Nitrosaminwerte erhalten werden wie nach den bekannten Verfahrensweisen der direkten Nitrosaminentfernung aus dem Trifluralin. Wenn das 4-Chlor-3,5-dinitrobenzotrifluorid nur mit Wasser — also in Abwesenheit einer anorganischen Base — bei erhöhter Temperatur unter Hindurchleiten von Inertgas behandelt wird, wird im Trifluralin keine oder praktisch keine Erniedrigung des Nitrosaminwertes erzielt (siehe die tabellarische Zusammenstellung der Versuchsergebnisse auf Seite 16 der DE-OS 2 840 551). Das Verfahren zur Entfernung von Nitrosierungsmittel(n) aus der Trifluoralin-Vorstufe gemäß DE-OS 2 840 551 erscheint zwar — weil im Trifluralin dann kaum mehr etwa ein Wiederanstieg des Nitrosaminwertes zu befürchten ist — günstiger als die Verfahrensweisen der DE-OSen 2 831 119, 2 835 530 und 2 837 529, doch ist insbesondere die Notwendigkeit von gegebenenfalls mehreren Arbeitsgängen auch hier nachteilig.

Es bestand daher die Aufgabe, unter Vermeidung der Nachteile der bekannten Verfahrensweisen ein einfacheres und wirtschaftliches Verfahren zu entwickeln, welches die Herstellung eines nitrosaminfreien Trifluralins gestattet — sei es durch Entfernung der Nitrosamine in dem fertigen Trifluralin oder durch Vermeidung der Bildung dieser Nitrosamine — und welches sich auch über diesen Spezialfall hinaus möglichst allgemein anwenden läßt.

Diese Aufgabe wurde erfindungsgemäß in einfacher und befriedigender Weise im Rahmen einer Wasserbehandlung des 4-Chlor-3,5-dinitrobenzotrifluorids (bei normaler oder bei erhöhter Temperatur) dadurch gelöst, daß man das hierbei verwendete Wasser zumindest teilweise wieder dampfförmig entfernt. Das Verfahren läßt sich über diesen Spezialfall hinaus auch auf andere nitrierte aromatische Verbindungen zur Entfernung der darin von der Nitrierung her noch enthaltenen Nitrierungsmittel anwenden.

Es war außerordentlich überraschend, daß durch die zumindest teilweise Entfernung des bei der Wasserbehandlung nitrierter aromatischer Verbindungen verwendeten Wassers im Dampfzustand eine Entfernung der Nitrierungsmittel erfolgt, weil man insbesondere aufgrund der tabellarischen Zusammenstellung auf Seite 16 der DE-OS 2 840 551 kaum annehmen konnte, daß in nitrierten aromatischen Verbindungen von der Nitrierung her noch vorhandene Nitrierungsmittel durch eine Wasserbehandlung ohne Zusatz weiterer Stoffe (z. B. basischer Stoffe) zu entfernen sind.

Erfindungsgegenstand ist somit ein Verfahren zur Entfernung von Nitrosierungsmittel(n) aus nitrierten aromatischen Verbindungen durch eine Wasserbehandlung, das dadurch gekennzeichnet ist, daß man das für die Wasserbehandlung verwendete Wasser zumindest teilweise wieder dampfförmig entfernt.

Als dem Verfahren zugängliche nitrierte aromatische Verbindungen kommen im Prinzip alle möglichen nitrierten aromatischen Verbindungen der eingangs gegebenen Definition infrage, also z. B. Nitrobenzol, Nitronaphthalin und deren Derivate etc. Bevorzugt sind jedoch nitrierte aromatische Verbindungen der folgenden allgemeinen Formel I:

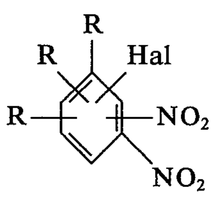

(I)

worin die Reste R unabhängig voneinander

$$= H, (C_1—C_4)\text{-Alkyl}, CF_3, SO_2NH_2, SO_2CH_3,$$

und

$$Hal = F, Cl, Br, J, \text{vorzugsweise } Cl, Br.$$

Konkrete unter diese Formel fallende Verbindungen sind z. B.:

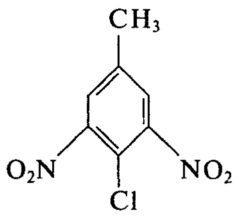
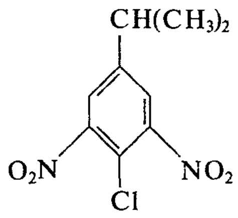
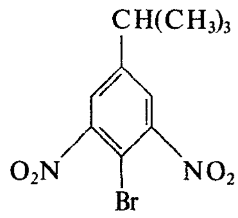

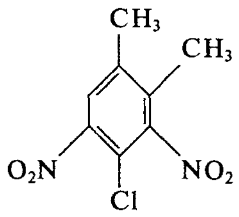
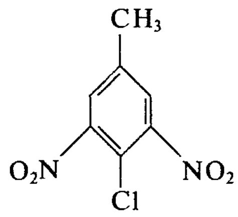
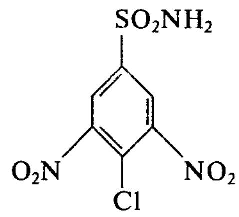

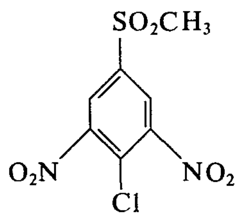

etc.

Die für das Verfahren besonders bevorzugte Ausgangsverbindung ist das Trifluralin-Vorprodukt 4-Chlor-3,5-dinitrobenzotrifluorid.

Die erfindungsgemäße Wasserbehandlung mit gleichzeitiger oder nachfolgender mindestens teilweiser Entfernung des Wassers im Dampfzustand erfolgt in einer bevorzugten Ausführungsform im allgemeinen bei Temperaturen zwischen 80 und 120° C, vorzugsweise zwischen 95 und 100° C. Die dafür eingesetzte Wassermenge kann im Prinzip beliebig sein, doch ist es bevorzugt, die Wassermenge zu 10 bis 50 Gew.-%, vorzugsweise zu 20 bis 40 Gew.-%, bezogen auf die nitrierte aromatische Ausgangsverbindung, zu bemessen. Die erfindungsgemäße Wasserbehandlung wird in dieser Ausführungsform vorteilhaft in der Weise durchgeführt, daß man Wasserdampf durch die nitrierte aromatische Ausgangsverbindung hindurchleitet (wie bei einer Wasserdampfdestillation) oder daß man die nitrierte aromatische Ausgangsverbindung mit Wasser auf eine Temperatur in dem genannten Bereich erhitzt und das Wasser dann zumindest teilweise — bevorzugt unter schwachem Vakuum bei 95 bis 100° C — abdestilliert. Die nitrierte aromatische Verbindung soll hier bevorzugt in flüssiger bzw. geschmolzener Form vorliegen.

Die Zeitdauer für diese Wasserbehandlung mit zumindest teilweiser Entfernung des Wassers im Dampfzustand liegt zweckmäßig zwischen 10 und 60 Minuten; unter- und oberhalb dieses Zeitbereiches liegende Behandlungszeiten sind je nach der Ansatzgröße und den Behandlungsbedingungen im einzelnen (Temperatur, Geschwindigkeit der Wasserdampfdurchleitung oder der Abdestillation des Wassers etc.) möglich, im allgemeinen jedoch ohne besonderen Vorteil.

In einer anderen bevorzugten, noch schonenderen Ausführungsform, wird die Wasserbehandlung in Form einer Kristallisation aus wäßriger Lösung oder Emulsion unter vermindertem Druck, und zwar auch bei Temperaturen bis hinab zur Raumtemperatur, durchgeführt.

Von der nach der Kristallisation aus wäßriger Lösung oder Emulsion unter vermindertem Druck erhaltenen wäßrigen Kristallsuspension wird dann weiterhin mit Vorteil noch zumindest ein Teil des Wassers ebenfalls unter vermindertem Druck abgezogen.

Entgegen der zuerst beschriebenen Ausführungsform des erfindungsgemäßen Verfahrens (Durchführungs praktisch nur an flüssigen bzw. geschmolzenen nitrierten aromatischen Verbindungen) arbeitet die zuletzt geschilderte Ausführungsform im Kristallisationsbereich der entsprechenden nitrierten aromatischen Verbindungen — wenn diese also vom flüssigen in den festen Zustand übergehen — sowie wenn die nitrierten aromatischen Verbindungen bereits fest sind.

Für diese Ausführungsform ist wesentlich, daß während der Kristallisation aus wäßriger Lösung oder Emulsion ein verminderter Druck, welcher eine Abdestillation des Wassers erlaubt, herrscht. Dieser Druck muß daher unterhalb des Dampfdrucks des Wassers bei der entsprechenden Temperatur liegen. Wenn noch größere Wassermengen vorhanden sind, ist es zweckmäßig, wenn der angewandte verminderte Druck den Wasserdampfdruck bei der jeweiligen Temperatur nicht zu sehr unterschreitet, damit nicht etwa unerwünschte Siedeverzüge oder dergleichen entstehen.

Das gleiche bezüglich des anzuwendenen verminderten Drucks gilt auch für das Abziehen des Wassers aus der entsprechenden wäßrigen Kristallsuspension.

Unter Beachtung der hier beschriebenen Kriterien kann der Druck bis herab zu etwa 10 mbar betragen. Es muß lediglich immer eine ausreichende Wasser-Verdampfungsgeschwindigkeit und ein störungsfreies Verdampfen gewährleistet sein.

Es versteht sich von selbst, daß damit dieser Ausführungsform des Verfahrens natürlich keine solchen nitrierten aromatischen Verbindungen zugänglich sind, welche etwa einen höheren Dampfdruck als das Wasser bei den jeweiligen Temperaturen besitzen.

Die Temperaturen der zuletzt genannten Ausführungsform der Erfindung liegen bei den Temperaturen der zuerst beschriebenen Verfahrensweise, bis herab etwa zur Raumtemperatur (etwa 20° C), also zwischen etwa 20 bis 120° C. Natürlich hängen die Temperaturen im Einzelfall vom Schmelzpunkt der jeweiligen nitrierten aromatischen Verbindung ab; sie bewegen sich um diesen Schmelzpunkt und darunter. Damit liegen die Temperaturen für die Behandlung ein und derselben nitrierten aromatischen Verbindungen hier niedriger als bei der erstgenannten Ausführungsform, bei welcher sich die nitrierten aromatischen Verbindungen praktisch immer nur in flüssigem Zustand befinden. Unter ca. 20° C liegende Temperaturen wären im Prinzip für die erfindungsgemäße Wasserbehandlung möglich, bringen jedoch keine Vorteile.

Durch die erfindungsgemäße Wasserbehandlung mit zumindest teilweiser Entfernung des Wassers im Dampfzustand werden die in den nitrierten aromatischen Ausgangsverbindungen von der Nitrierung her als Nebenprodukte vorhandenen Nitrosierungsmittel wahrscheinlich gespalten, zersetzt und/oder mit dem Wasserdampf entfernt.

Wenn die so behandelten nitrierten aromatischen Verbindungen etwa mit Aminen weiter umgesetzt werden, lassen sich in den Umsetzungsprodukten praktisch keine Nitrosamine mehr nachweisen.

Von den bekannten Verfahrensweisen der Entfernung der Nitrosamine aus den entsprechenden Umsetzungsprodukten sowie der Entfernung von Nitrosierungsmittel(n) aus den nitrierten aromatischen Verbindungen unterscheidet sich das erfindungsgemäße Verfahren vorteilhaft vor allem dadurch, daß es auf einfachste Weise ohne Einsatz zusätzlicher Chemikalien und Verfahrensstufen durchführbar ist; da nach der Nitrierung im allgemeinen sowieso eine Neutralwäsche zur Reinigung der nitrierten aromatischen Verbindung durchgeführt wird, kann die erfindungsgemäße Entfernung von Nitrosierungsmittel(n) praktisch im gleichen Verfahrensgang mit erfolgen. Die an zweiter Stelle beschriebene Ausführungsform des Verfahrens ist gegenüber der zuerst geschilderten wegen der tieferen Temperaturen noch etwas schonender.

Die Erfindung stellt daher einen erheblichen Fortschritt dar.

Die folgenden Beispiele sollen nun der näheren Erläuterung der Erfindung dienen. Die Nitrosaminkonzentration wurde nach den z. B. in der DE-OS 2 831 119 angegebenen Methoden bestimmt.

## Beispiel 1

a) Herstellung von Trifluralin auf übliche Weise:
2 Mol p-Chlorbenzotrifluorid wurden in bekannter Weise zunächst mononitriert, alsdann bei 110 bis 125° C dinitriert. Nach Verdünnen der Endsäure auf eine ca. 70gewichtsprozentige Schwefelsäure wurde bei 80° C das Nitroprodukt flüssig abgetrennt und mehrmals mit Wasser und etwas Natronlauge neutral gewaschen.

Ausbeute an 4-Chlor-3,5-dinitrobenzotrifluorid: 432 g EP.: 54,8° C

Nach üblicher Umsetzung mit Di-n-propylamin zum Trifluralin (Ausbeute: praktisch quantitativ, EP. 46,2° C) wurde in diesem ein Nitroso-di-n-propylamingehalt von 29 ppm ermittelt.

b) Erfindungsgemäße Entfernung von Nitrosierungsmittel(n) aus dem Vorprodukt 4-Chlor-3,5-dinitrobenzotrifluorid nach der ersten bevorzugten Ausführungsform der Erfindung: In 500 g 4-Chlor-3,5-dinitrobenzotrifluorid (nach Beispiel 1a hergestellt) wurde bei ca. 220 g Kondensat anfiel. Anschließend erfolgte die übliche Umsetzung zum Trifluralin; Nitroso-di-n-propylamingehalt: 1 ppm.

c) Erfindungsgemäße Entfernung von Nitrosierungsmittel(n) aus dem gleichen Vorprodukt nach der anderen bevorzugten Ausführungsform der Erfindung: 500 g 4-Chlor-3,5-dinitrobenzotrifluorid (hergestellt wie in Beispiel 1a beschrieben) wurden unter gutem Rühren bei 70° C in 500 ml Wasser emulgiert, mittels teilweiser Vakuumverdampfung des Wassers auf 50° C abgekühlt und bei dieser Temperatur kristallisiert.

Nach weiterem Abkühlen auf etwa 40° C wurde bei 40 bis 50° C unter vermindertem Druck so lange nachgeheizt, bis insgesamt etwa 100 ml Wasser abdestilliert waren.

Nach dem Aufschmelzen und dem Abtrennen des Wassers (etwa durch Dekantieren) wurde bei der wie üblich durchgeführten Umsetzung mit Di-n-propylamin zu Trifluralin ein Produkt mit einem Nitroso-di-n-propylamingehalt von 1 ppm erhalten.

Beispiel 2

500 g 4-Chlor-3,5-dinitrobenzotrifluorid (nach Beispiel 1a hergestellt) und 220 g Wasser wurden auf 98 bis 100°C erhitzt. Darauf wurde unter schwachem Vakuum das gesamte Wasser abdestilliert. Anschließend erfolgte die übliche Umsetzung zum Trifluralin, Nitroso-di-n-propalamingehalt: 2 ppm.

**Patentansprüche**

1. Verfahren zur Entfernung von Nitrosierungsmittel(n) aus nitrierten aromatischen Verbindungen durch eine Wasserbehandlung, dadurch gekennzeichnet, daß man das für die Wasserbehandlung verwendete Wasser zumindest teilweise wieder dampfförmig entfernt.

2. Verfahren nach Anspruch 1, dadurch gekennzeichnet, daß man als nitrierte aromatische Verbindungen der Formel I

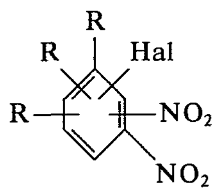

(I)

worin die Reste R unabhängig voneinander

$$= H, (C_1-C_4)\text{-Alkyl}, CF_3, SO_2NH_2, SO_2CH_3,$$

und

$$Hal = F, Cl, Br, J, \text{vorzugsweise } Cl, Br.$$

verwendet.

3. Verfahren nach den Ansprüchen 1 und 2, dadurch gekennzeichnet, daß man als nitrierte aromatische Verbindung 4-Chlor-3,5-dinitrobenzotrifluorid verwendet.

4. Verfahren nach den Ansprüchen 1 bis 3, dadurch gekennzeichnet, daß man die Wasserbehandlung bei Temperaturen von 80 bis 120° C, vorzugsweise von 95 bis 100° C, durchführt.

5. Verfahren nach den Ansprüchen 1 bis 4, dadurch gekennzeichnet, daß man für die Wasserbehandlung eine Wassermenge von 10 bis 50 Gew.-%, vorzugsweise von 20 bis 40 Gew.-%, bezogen auf die nitrierte aromatische Verbindung, verwendet.

6. Verfahren nach den Ansprüchen 1 bis 5, dadurch gekennzeichnet, daß man die Wasserbehandlung in der Weise durchführt, daß man Wasserdampf durch die nitrierte aromatische Verbindung leitet.

7. Verfahren nach den Ansprüchen 1 bis 5, dadurch gekennzeichnet, daß man die Wasserbehandlung in der Weise durchführt, daß man die nitrierte aromatische Verbindung mit Wasser erhitzt und das Wasser gegebenenfalls unter schwachem Vakuum bei Temperaturen zwischen 95 und 100° C abdestilliert.

8. Verfahren nach den Ansprüchen 1 bis 7, dadurch gekennzeichnet, daß man die Wasserbehandlung während einer Zeitdauer von 10 bis 60 Minuten durchführt.

9. Verfahren nach den Ansprüchen 1 bis 3, dadurch gekennzeichnet, daß man die Wasserbehandlung in Form einer Kristallisation aus wäßriger Lösung oder Emulsion unter vermindertem Druck durchführt.

10. Verfahren nach Anspruch 9, dadurch gekennzeichnet, daß man von der nach der Kristallisation aus wäßriger Lösung unter vermindertem Druck erhaltenen Kristallsuspension noch zumindest einen Teil des Wassers ebenfalls unter vermindertem Druck abzieht.

**Claims**

1. Method for removing nitrosation agent(s) from nitrated aromatic compounds by a water treatment, characterized in removing at least partially in vapor form the water used for the treatment.

2. Method as claimed in claim 1, characterized in using compounds of the formula I

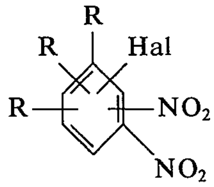

(I)

in which the radicals R, independent of one another, are hydrogen, $C_1-C_4$ alkyl, $CF_3$, $SO_2NH_2$, $SO_2CH_3$ and Hal denotes F, Cl, Br, I, preferably Cl and Br, as nitrated aromatic compounds.

3. Methods as claimed in claims 1 to 2, characterized in using 4-chloro-3,5-dinitrobenzotrifluoride as nitrated aromatic compound.

4. Method as claimed in claims 1 to 3, characterized in carrying out the water treatment at temperatures of from 80 to 120° C, preferably 95 to 100° C.

5. Method as claimed in claims 1 to 4, characterized in using 10 to 50% by weight, preferably 20 to 40% by weight of water, calculated on the nitrated aromatic compound, for the water treatment.

6. Method as claimed in claims 1 to 5, characterized in carrying out the water treatment by passing steam through the nitrated aromatic compound.

7. Method as claimed in claims 1 to 5, characterized in carrying out the water treatment by heating the nitrated aromatic compound together with water and distalling off the water at a temperature of from 95 to 100° C, optionally under slightly reduced pressure.

8. Method as claimed in claims 1 to 7, characterized in carrying out the water treatment for 10 to 60 minutes.

9. Method as claimed in claims 1 to 3, characterized in carrying out the water treatment in the form of a crystallization from aqueous solution or emulsion under reduced pressure.

10. Method as claimed in claim 9, characterized in removing from the crystal suspension obtained after crystallization from aqueous solution under reduced pressure at least part of the water under reduced pressure.

**Revendications**

1. Provédé pour éliminer, par un traitement à l'eau, un ou des agents de nitrosation contenus dans des composés aromatiques nitrés, procédé caractérisé en ce que l'eau utilisée pour ledit traitement à l'eau est éliminée à nouveau au moins partiellement à l'état de vapeur.

2. Procédé selon la revendication 1, caractérisé en ce qu'on utilise comme composés aromatiques nitrés, des composés répondant à la formule I

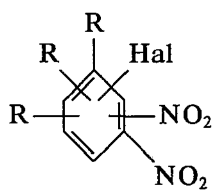

(I)

dans laquelle

| | |
|---|---|
| les R | représentent chacon, indépendamment les uns des autres, H, un alkyle en $C_1-C_4$, $CF_3$, $SO_2NH_2$ ou $SO_2CH_3$ et |
| Hal | représente F, Cl, Br ou I, de préference Cl ou Br. |

3. Procédé selon l'une des revendications 1 et 2, caractérisé en ce qu'on utilise, comme composé aromatique nitré, le trifluorométhyl-1 dinitro-3,5 chloro-4 benzène.

4. Procédé selon l'une quelconque des revendications 1 à 3, caractérisé en ce que le traitement par l'eau est effectué à des températures de 80 à 120° C, de préférence de 95 à 100° C.

5. Procédé selon l'une quelconque des revendications 1 à 4, caractérisé en ce qu'on utilise, pour le traitement à l'eau, une quantité d'eau de 10 à 50% en poids, de préférence de 20 à 40% en poids, par rapport au composé aromatique nitré.

6. Procédé selon l'une quelconque des revendications 1 à 5, caractérisé en ce que, pour effectuer le traitement par l'eau, on fait passer de la vapeur d'eau dans le composé aromatique nitré.

7. Procédé selon l'une quelconque des revendications 1 à 5, caractérisé en ce que, pour effectuer le traitement par l'eau, on chauffe le composé aromatique nitré avec de l'eau et on chasse l'eau par distillation, éventuellement sous un léger vide, à des températures comprises entre 95 et 100° C.

8. Procédé selon l'une quelconque des revendications 1 à 7, caractérisé en ce qu'on effecture le traitement par l'eau pendant une durée de 10 à 60 minutes.

9. Procédé selon l'une quelconque des revendications 1 à 3, caractérisé en ce que le traitement par l'eau est effectué sous la forme d'une cristallisation dans une solution ou émulsion aqueuse, sous pression réduite.

10. Procédé selon la revendication 9, caractérisé en ce qu'on chasse encore au moins une partie de l'eau, également sous pression réduite, de la suspension de cristaux qui a été obtenue après la cristallisation dans la solution aqueuse sous pression réduite.